# FASCICULE DE BREVET EUROPEEN

(11) **EP 4 336 181 B1**
(45) Date de publication et mention de la délivrance du brevet: **18.06.2025**
(21) Numéro de dépôt: 24153553.3
(22) Date de dépôt: 03.03.2021
(51) Int. Cl.: A61B 10/00, A61B 5/145, G01N 33/493, G01N 33/52, A61B 5/20, G01N 33/62, A61B 5/00

(54) **DISPOSITIF ET MÉTHODE D'ANALYSE D'URINE**
URINANALYSEVORRICHTUNG UND -VERFAHREN
URINE ANALYSIS DEVICE AND METHOD

(30) Priorité: 03.03.2020 FR 2002125
(43) Date de publication de la demande: 13.03.2024
(62) Demande divisionnaire de: 21708250.2
(73) Titulaire: Withings, 92130 Issy-Les-Moulineaux (FR)
(72) Inventeur: Barbedette, Thibaut, 92130 Issy-les-Moulineaux (FR); Kirat, Marine, 92130 Issy-les-Moulineaux (FR); Loy, Jonas, 92130 Issy-les-Moulineaux (FR); Tucoulat, Benoît, 92130 Issy-les-Moulineaux (FR); Dewavrin, Julius, 92130 Issy-les-Moulineaux (FR); Barakat, Christelle, 92130 Issy-les-Moulineaux (FR)
(74) Mandataire: Withings IP

(56) Documents cités:
- EP-A1- 3 156 550
- WO-A1-2011/140600
- US-A1- 2005 261 605
- US-A1- 2017 022 536
- US-A1- 2019 062 813

## Description

### Domaine technique

La présente divulgation relève du domaine des dispositifs d'analyse d'urine destinés à être positionnés à l'intérieur de toilettes. La présente divulgation vise également une méthode pour analyser l'urine étant reçue dans des toilettes.

### Technique antérieure

De nombreux paramètres biologiques sont reflétés dans l'urine d'un individu. A partir d'un échantillon d'urine, il est par exemple possible de détecter des problèmes de santé tels qu'une infection urinaire, le diabète ou une insuffisance rénale. L'échantillon d'urine peut également refléter la qualité d'une alimentation, identifier une période de fécondité ou une grossesse et détecter une prise de drogues ou de tabac. Il est alors intéressant de surveiller divers paramètres biologiques périodiquement.

Il est connu de proposer des dispositifs installés aux toilettes ayant une fonction d'analyse d'urine. Ces dispositifs sont capables d'effectuer des prélèvements d'échantillons d'urine depuis les toilettes et de les analyser afin de déterminer le niveau d'un paramètre biologique.

Il est décrit dans les documents US5720054 et US10383606 de prélever un échantillon d'urine au travers d'un bras mobile situé dans les toilettes. Néanmoins. cette solution présente la difficulté de collecter suffisamment d'urine sans bulles d'air pour réaliser une analyse concluante. De plus, l'usage d'un bras mobile ajoute de la complexité au dispositif.

On connait du document US20180188231 un dispositif d'analyse d'urine à fixer sur un rebord de toilettes. Cependant, il apparaît encore possible d'améliorer la collecte d'urine.

Le document US20170284925 propose d'intégrer un préleveur d'urine dans la cuvette des toilettes. Néanmoins, cette solution nécessite d'adapter les toilettes pour l'installation et l'utilisation du dispositif. Le document US2019/0062813 décrit un système de toilettes intelligentes pour analyser des déchets biologiques, et est considéré comme l'état de la technique le plus proche de l'invention. Le document US2005/0261605 décrit un système d'analyse médical pour mesurer des propriétés de l'urine. Le document US2017/0022536 décrit un système de mesure et d'analyse des niveaux de métabolites dans l'urine.

Il existe donc un besoin pour un dispositif d'analyse d'urine pouvant prélever un échantillon d'urine depuis les toilettes ne présentant pas les inconvénients de l'art antérieur

### Exposé de l'invention

La présente description vise à présenter des dispositifs d'analyse d'urine et des méthodes associées ne présentant pas au moins un des inconvénients précités. L'invention est définie par les revendications.

En particulier, il est proposé un dispositif d'analyse d'urine comprenant un boitier configuré pour être entièrement positionné à l'intérieur de toilettes, le boitier présentant une face avant destinée à recevoir un jet d'urine directement d'un utilisateur urinant en position assise sur les toilettes, une face arrière opposée à la face avant, et un orifice de collecte, disposé sur la face arrière, dans lequel le boitier contient un ensemble de test configuré pour effectuer une analyse sur l'urine collectée par l'orifice de collecte.

Ainsi, avantageusement, le dispositif d'analyse d'urine peut collecter de l'urine par l'orifice de collecte directement par ruissèlement sur les faces du boitier. Un utilisateur n'a pas besoin de de se préoccuper de la position du boitier lorsqu'il urine dans les toilettes. En outre, le boitier est suffisamment compact pour être entièrement positionné à l'intérieur des toilettes. Il peut être discret et être installé et retiré facilement. Il peut également s'adapter à tout type de toilettes. En outre, le boitier ne présente pas de pièces saillantes désagréables à la vue l'utilisateur et qui peuvent accumuler de la saleté. L'aspect général monobloc ou monolithique du boîtier lui confère une image de robustesse et de simplicité.

La collecte d'urine est facilitée et l'utilisateur n'a juste qu'à réaliser une miction sans vraiment se préoccuper du positionnement du dispositif.

Les caractéristiques exposées dans les paragraphes suivants peuvent, optionnellement, être mises en œuvre. Elles peuvent être mises en œuvre indépendamment les unes des autres ou en combinaison les unes avec les autres.

L'orifice de collecte peut être situé au voisinage d'une extrémité inferieure du boitier en position normale d'utilisation. Par exemple, l'orifice de collecte peut être situé sur une moitié inférieure du boitier en position normale d'utilisation. Ainsi, de l'urine ruisselant par gravité sur les faces du boitier peut être collectée. La surface de collecte d'urine est augmentée, il suffit qu'un peu d'urine atteigne le boîtier pour qu'elle dégouline le long des parois du boîtier et atteigne l'orifice de collecte.

Le boitier peut être dépourvu d'arête. Moyennant quoi, de l'urine peut ruisseler sur le boitier sans décrocher ou former de bulles d'air.

La face avant et/ou la face arrière peut comprendre un relief formant une amenée d'urine vers l'orifice de collecte. Quel que soit l'endroit où de l'urine entre en contact avec le boitier, il sera acheminé vers l'orifice de collecte. Ainsi, la surface de collecte d'urine est augmentée. Le relief peut être négatif (de type renfoncement) ou positif (de type saillie). Le relief est par exemple formé dans le boitier (la face avant et/ou la face arrière).

L'orifice de collecte est situé dans un renfoncement, ayant une bordure, le renfoncement comportant deux rainures latérales s'étendant depuis un bord du boitier vers une partie centrale du renfoncement, la partie centrale du renfoncement logeant l'orifice de collecte. La profondeur des rainures latérales peut croitre depuis le bord jusqu'à la partie centrale. La bordure peut présenter une inflexion entre au moins une rainure latérale et la partie centrale. La distance entre la bordure du renfoncement et le bord du boitier peut croitre depuis le début de la rainure latérale jusqu'à la partie centrale. La partie centrale peut avoir une superficie comprise entre 3 et 8 cm².

Le renfoncement (ou la partie centrale du renfoncement) peut s'étendre vers l'intérieur du boitier sur une distance comprise entre 1 et 4cm.

Le boitier peut avoir une forme générale de galet circulaire. Ainsi, la forme du boitier est définie par des surfaces courbées, et constitue une forme simple et naturelle agréable à la vue de l'utilisateur. De l'urine peut ruisseler sur tout le boitier sans décrocher ou former de bulles d'air.

Le dispositif d'analyse d'urine peut être dépourvu de pièces mobiles à l'extérieur du boitier. Le dispositif d'analyse d'urine ne nécessite pas de joint. Le boitier est dépourvu de points de stagnation ou de l'urine pourrait stagner. Le dispositif d'analyse d'urine est hygiénique.

Le boitier peut comprendre un orifice de vidange configuré pour évacuer l'urine. Moyennant quoi, un excès d'urine collecté peut être purgé du dispositif d'analyse d'urine. Une collecte d'urine n'est alors pas contaminée par une collecte précédente. Plusieurs collectes d'urine consécutives peuvent être réalisées.

L'orifice de vidange et l'orifice de collecte peuvent être équipés d'un filtre en maille métallique. Ainsi, le filtre peut empêcher l'introduction de contaminants ou d'éléments susceptibles d'obstruer le système de test, et de filtrer l'urine avant d'atteindre l'orifice de collecte.

Le boitier peut être en matériau hydrophile, de préférence le matériau hydrophile est un parmi : une céramique, un polyamide, un silicone, un polymère hydrophile, et/ou le boitier peut être traité par un traitement de surface hydrophile. Ainsi, de l'urine entrant en contact avec le boitier accroche et s'étale sur le boitier.

Le diamètre du boitier peut être compris entre 50 mm et 150 mm, de préférence le diamètre peut être compris entre 80 mm et 120 mm, et de manière encore plus préférée, le diamètre du boitier est voisin de 100 mm. Le boitier est alors suffisamment compact pour être entièrement reçu dans les toilettes. Le boitier est discret. Le boitier est également suffisamment étendu pour systématiquement entrer en contact avec de l'urine étant reçu dans les toilettes.

Le boitier peut être constitué d'une coque avant et d'une coque arrière. La coque avant et la coque arrière peuvent être jointes par vissage, collage, clipsage, par aimantation, ou soudure ultrason. Alors, l'assemblage du boitier est facilité. En outre, la jointure entre la coque avant et la coque arrière peut permettre le ruissellement d'urine entre la coque avant et la coque arrière.

Le dispositif d'analyse d'urine peut comporter un capteur de présence d'urine, au voisinage de l'orifice de collecte, le capteur étant configuré pour détecter la présence d'urine, de préférence le capteur étant un capteur de température. Ainsi, le dispositif d'analyse d'urine peut déclencher une analyse lorsque de l'urine est détecté sur le boitier.

Le capteur de présence d'urine peut être un capteur de température. Le capteur de température permet de distinguer entre de l'urine et de l'eau étant détectée sur le boitier. En outre, le capteur de température peut détecter une période de fécondité. Ainsi, le capteur de température peut à la fois détecter la présence d'urine et réaliser une analyse. Le nombre de composants du dispositif d'analyse d'urine est réduit.

Le dispositif d'analyse d'urine peut comporter un élément de fixation configuré pour positionner le boitier sur une paroi interne d'une cuvette de toilettes. Alors, le dispositif d'analyse d'urine peut être déplacé ou retiré des toilettes.

Le dispositif d'analyse d'urine peut comporter un module de communication avec un appareil et/ou un serveur et/ou un smartphone. Ainsi, le dispositif d'analyse d'urine peut être commandé pour déclencher une analyse. Le dispositif d'analyse d'urine peut analyser et transmettre un ou plusieurs résultats d'analyses.

L'appareil peut comprendre un bouton. Moyennant quoi, une analyse peut être déclenchée lorsqu'un utilisateur appuie sur le bouton.

Le bouton peut être pourvu d'un capteur biométrique. Moyennant quoi, l'utilisateur peut être identifié et l'analyse ne s'enclencher que si un utilisateur est identifié. Une analyse peut être adaptée à l'utilisateur identifié.

L'ensemble de test peut comporter un ou plusieurs parmi : une pluralité de bandelettes de test, une puce micro-fluidique, un transistor à effet de champ, un dispositif de mesure de conductivité, un dispositif de mesure de pH, un dispositif de mesure spectroscopique, un dispositif de mesure électrochimique. Ainsi, un même boitier peut être adapté pour analyser divers paramètres biologiques contenus dans l'urine. Le dispositif d'analyse d'urine est polyvalent.

Selon un autre aspect, il est proposé une **méthode** de collection d'urine exploitant le dispositif d'analyse d'urine comprenant :
- détecter la présence d'urine à proximité de l'orifice de collecte
- collecter l'urine ruisselant sur la face avant et/ou la face arrière,
- analyser l'urine collectée pour établir un ou plusieurs résultats d'analyses.

Les caractéristiques exposées dans les paragraphes suivants peuvent, optionnellement, être mises en œuvre. Elles peuvent être mises en œuvre indépendamment les unes des autres ou en combinaison les unes avec les autres.

La méthode peut être déclenchée par une interaction avec un utilisateur. La méthode peut être déclenchée par l'appui sur un bouton. La méthode peut être lancée par détection d'un utilisateur à proximité des toilettes. La méthode peut également être lancée lors de la détection d'urine sur le dispositif d'analyse d'urine.

Le ou les résultats d'analyses peuvent être transmis à un smartphone d'un utilisateur et/ou à un serveur distant.

### Brève description des dessins

D'autres caractéristiques, détails et avantages apparaîtront à la lecture de la description détaillée ci-après, et à l'analyse des dessins annexés, sur lesquels :
[Fig. 1] représente schématiquement une vue en coupe de toilettes équipées d'un dispositif d'analyse d'urine au sens de l'invention.
[Fig. 2] représente schématiquement un détail de la Figure 1.
[Fig. 3] illustre une vue en perspective d'un premier boitier, selon un premier mode de réalisation, pouvant être mis en œuvre dans le dispositif d'analyse d'urine de la figure 1.
[Fig. 4] illustre une vue en perspective éclatée du premier boitier de la figure 3 et d'un ensemble de test pouvant être mis en œuvre dans le dispositif d'analyse d'urine de la figure 1.
[Fig. 5] illustre une vue en perspective partiellement éclatée d'un deuxième boitier, selon un deuxième mode de réalisation, pouvant être mis en œuvre dans le dispositif d'analyse d'urine de la figure 1.
[Fig. 6] illustre un détail d'un troisième boitier, selon un troisième mode de réalisation, pouvant être mis en œuvre dans le dispositif d'analyse d'urine de la figure 1.
[Fig. 7] illustre une autre vue en perspective du premier boitier de la figure 3.
[Fig. 8] illustre schématiquement une vue de face d'un boitier selon un quatrième mode de réalisation, pouvant être mis en œuvre dans le dispositif d'analyse d'urine de la figure 1.
[Fig. 9] illustre schématiquement l'ensemble de test.
[Fig. 10] illustre schématiquement un organigramme selon un mode de réalisation.
[Fig. 11] illustre schématiquement un organigramme selon un autre mode de réalisation.
[Fig. 12] représente schématiquement un détail de la figure 1, selon un autre mode de réalisation.
[Fig. 13] représente une vue en perspective d'une face arrière du dispositif, selon un autre mode de réalisation.

### Description des modes de réalisation

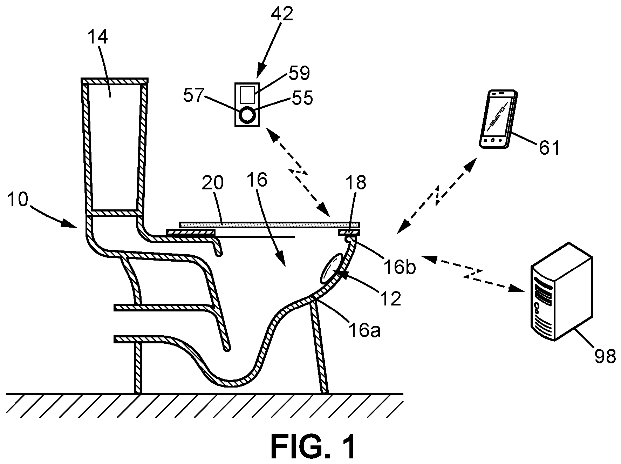

La figure 1 illustre des toilettes **10** équipées d'un dispositif d'analyse d'urine **12.** De manière connue, les toilettes comportent un réservoir d'eau **14,** une cuvette **16,** un siège **18** et un couvercle **20.** Le dispositif d'analyse d'urine **12** est agencé sur une paroi interne **16a** de la cuvette 16 des toilettes. Avantageusement, le dispositif d'analyse d'urine **12** est entièrement reçu dans la cuvette **16** des toilettes. En effet, le dispositif d'analyse d'urine **12** ne dépasse pas du rebord supérieur **16b** de la cuvette et ainsi il reste discret.

Ici, le dispositif d'analyse d'urine **12** est positionné sur la trajectoire d'un jet d'urine secrété par un utilisateur. Le dispositif d'analyse d'urine reçoit un jet d'urine lorsqu'un utilisateur urine en position assise dans les toilettes. La position du dispositif d'analyse d'urine est alors adaptée pour tout type d'utilisateur, de sexe masculin ou féminin, quel que soit son âge. L'utilisateur peut alors uriner dans les toilettes sans se préoccuper de la position du dispositif d'analyse d'urine.

Ici, le dispositif d'analyse d'urine **12** est également positionné sur la trajectoire d'une chasse d'eau provenant du réservoir **14.** Ainsi, le dispositif d'analyse d'urine peut être rincé lors de l'actionnement de la chasse d'eau. Le dispositif d'analyse d'urine est hygiénique.

Le dispositif d'analyse d'urine **12** comprend un boitier **22** renfermant un ensemble de test **24.** L'ensemble de test est destiné à analyser l'urine étant reçue dans le dispositif d'analyse d'urine.

Le boitier **22** est agencé dans la cuvette 16 des toilettes de manière amovible. Le dispositif d'analyse **12** peut alors être retiré ou repositionné dans les toilettes. Le dispositif d'analyse d'urine est discret. En outre, le dispositif d'analyse d'urine peut être retiré pour recharger une batterie **94** ou un consommable **44** de l'ensemble de test **24.**

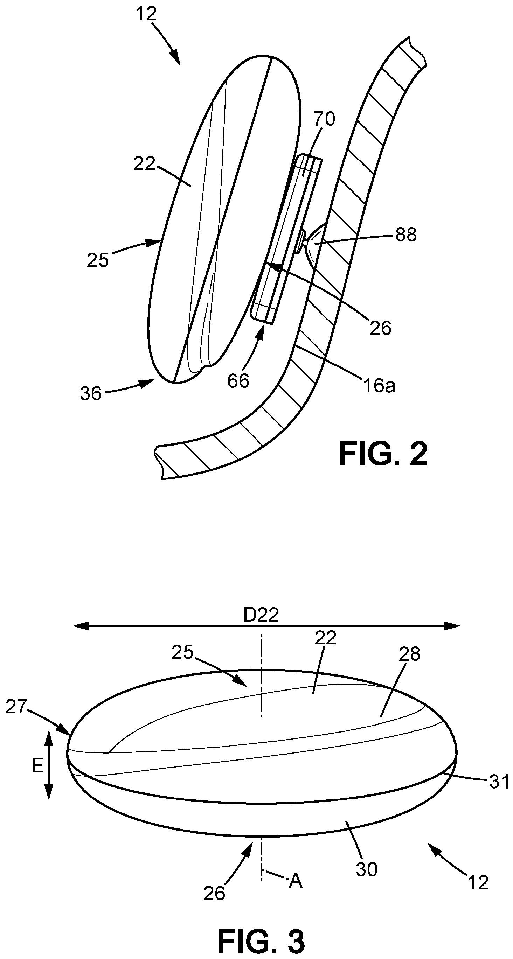

Dans l'exemple illustré à la figure 2, le boitier **22** est agencé sur la paroi **16a** des toilettes. Le boitier est positionné par un élément de fixation **66.** L'élément de fixation **66** comporte une ventouse **88** destinée à coopérer avec la paroi **16a** des toilettes et des aimants **70.** Les aimants **70** sont destinés à coopérer avec des aimants **23** (ou des pièces en matériau ferromagnétique) agencés à l'intérieur du boitier. Cette configuration permet de facilement retirer ou repositionner le boitier dans les toilettes.

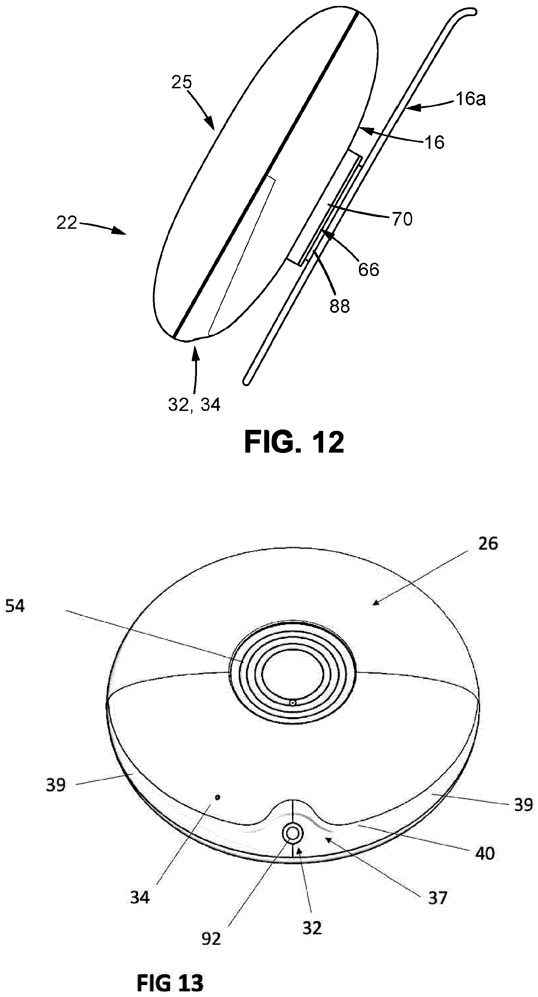

Dans l'exemple illustré à la figure 12, l'élément de fixation 66 est en outre de forme circulaire. L'élément de fixation 66 se loge dans un logement complémentaire prévu sur le boitier 22. Le logement peut être prévu au niveau de connecteurs de charge de la batterie 94 du dispositif d'analyse d'urine 12. Ainsi, les connecteurs de charge peuvent être protégés de l'eau des toilettes 10.

Des aides mécaniques, notamment des indentations, peuvent être prévues sur l'élément de fixation 66. Les aides mécaniques peuvent faciliter le positionnement du boitier 22 lors de son insertion dans les toilettes 10. La bonne position du boitier 22 dans les toilettes 10 assure le bon fonctionnement du dispositif d'analyse d'urine 12.

L'élément de fixation **66** peut également comprendre une liaison rotule. La liaison rotule permet d'orienter le boitier **22** afin d'augmenter la probabilité d'entrer en contact avec de l'urine étant reçue dans la cuvette **16** des toilettes.

En variante, l'élément de fixation **66** peut être un crochet monté sur un rebord **16b** de la cuvette **16** des toilettes.

### Caractéristiques générales du boitier

Le boitier **22** a une forme extérieure de galet circulaire. En d'autres termes, le boitier **22** a une forme de sphéroïde aplatie. Un axe **A** est l'axe médian du boitier. Le boitier **22** comporte une face avant **25** et une face arrière **26,** sensiblement normales à l'axe A. Avantageusement, la face avant **25** peut être substantiellement symétrique de révolution, ce qui donne un aspect épuré au dispositif une fois installé. Ainsi, une collecte d'urine peut se faire directement depuis les faces **25, 26** du boitier. Le boitier sert de collecteur d'urine.

La face avant **25** est orientée vers l'intérieur de la cuvette **16.** La face avant **25** est alors destinée à recevoir de l'urine lorsque l'utilisateur urine en position assise sur les toilettes **10.** La face arrière **26** est orientée face à la paroi intérieure 16a de la cuvette 16. La face avant **25** et la face arrière **26** sont reliées par des bords courbés **27.** Les bords courbés **27** respectifs de la face avant et la face arrière se rejoignent en affleurement au niveau d'une zone de jonction équatoriale. Alors, la surface extérieure du boitier **22,** constituée de la face avant **25,** la face arrière **26** et les bords courbés **27,** est définie par des lignes courbes, et forme un objet généralement convexe. Le boitier **22** est dépourvu d'arêtes. De l'urine peut ruisseler sur l'ensemble de la surface extérieure du boitier **22** sans décrocher du boitier ou former de bulles d'air, pouvant compromettre une analyse de l'urine.

Une arête peut être définie comme une transition abrupte entre deux surfaces. Une surface courbée peut être définie comme une surface dont la dérivée est continue et la dérivée seconde reste faible.

La surface extérieure du boitier 22 peut en outre être de couleur blanche ou claire. La couleur de la surface extérieure peut être similaire à celle des toilettes, augmentant la discrétion du dispositif.

De manière générale, le boitier **22** a un diamètre **D22,** mesuré dans la direction normale à l'axe **A,** compris entre 50 mm et 150 mm. On peut choisir de préférence un diamètre D22 compris entre 80 mm et 120 mm ; dans certains modes de réalisation, on peut choisir un diamètre voisin de 100 mm. Le boitier **22** a également une épaisseur **E,** mesuré dans la direction de l'axe **A,** comprise entre 15 mm et 50 mm, de préférence voisine de 30 mm. Ainsi, le boitier **22** est suffisamment compact pour être entièrement reçu dans la cuvette **16** des toilettes **10.** Le dispositif d'analyse d'urine **12** est discret. En outre, le boitier **22** est suffisamment étendu pour systématiquement entrer en contact avec de l'urine étant reçu dans la cuvette 16. L'utilisateur peut alors uriner dans les toilettes sans se soucier du dispositif d'analyse d'urine, ou à défaut viser sommairement.

Sous un autre point de vue, dans un mode de réalisation, le boitier présente un facteur de forme générale tel que le rapport **E/D22** soit compris dans l'intervalle [0,2 - 0,5] et voire de préférence compris dans l'intervalle [0,3 - 0,4] ; de telles proportions rappellent un galet naturel, et confère au dispositif un aspect apaisant.

La surface extérieure du boitier **22** est lisse. Ainsi, le jet d'urine entrant en contact avec le boitier **22** s'accroche et s'étale sur les surfaces extérieures du boitier. De manière préférée, le boitier **22** est d'un matériau hydrophile. Par exemple, la matière du boitier **22** peut être en un parmi : une céramique, un polyamide (PA), un silicone ou un polymère hydrophile. La surface extérieure du boitier **22** peut également être traitée par un traitement de surface hydrophile, par exemple acuWet^{®} de la société Aculon, un polymère hydrophile, ou Pebax^{®} de la société Arkema.

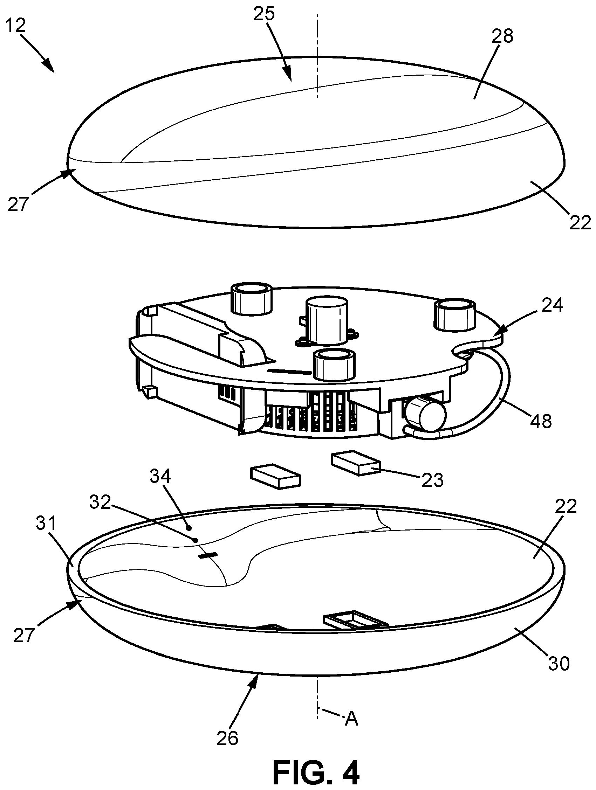

Comme plus visible sur la figure 4, selon une réalisation particulière, le boitier **22** est formé comme un assemblage de deux demi-coques et est constitué d'une coque avant **28** et d'une coque arrière **30.** La coque avant **28** et la coque arrière **30** forment une jointure annulaire **31** (également désigné bord 31) du boitier **22,** dans un plan normal à l'axe **A.** L'assemblage du dispositif d'analyse d'urine **12** est facilité lorsque le boitier **22** est constitué de la coque avant **28** et de la coque arrière **30.**

La coque avant **28** et la coque arrière **30** sont assemblées pour maintenir la surface extérieure du boitier **22** définie par des lignes courbes. Alors, la jointure annulaire **31** entre la coque avant et la coque arrière permet le ruissellement d'urine entre la face avant **25** et la face arrière **26.** L'impact de la jointure **31** sur l'écoulement d'urine sur le boitier est minimisé.

La coque avant **28** et la coque arrière **30** peuvent être assemblées par vissage, collage, clipsage, par aimantation, ou soudure ultrason. Bien entendu, d'autres moyens de fixation peuvent être mis en œuvre pour assembler la coque avant **28** et la coque arrière **30.**

Par exemple, la coque avant **28** et la coque arrière **30** sont assemblées par vissage. Alors, une portion interne de la coque avant **28** comporte un filetage. Le filetage de la coque avant **28** est destiné à coopérer avec un filetage complémentaire de la coque arrière **30.** Le boitier **22** peut ainsi facilement être démonté pour accéder à l'ensemble de test **24** à l'intérieur du boitier.

Dans un autre exemple, une portion interne de la coque arrière 30 comporte un filetage 140 destiné à coopérer avec un taraudage de la coque avant 28. On a un assemblage des deux coques 28, 30 par vissage. En alternative, l'assemblage des deux coques 28, 30 peut être un système à baïonnette.

Un joint d'étanchéité peut être présent au niveau de la jointure **31** entre la coque avant **28** et la coque arrière **30.** Ainsi, le boîtier **22** est étanche. L'intérieur du boitier **22** est imperméable à l'urine, à l'eau provenant du réservoir d'eau **14** ou de la cuvette **16,** et tout autre type de contaminant. Seuls des orifices de collecte et de vidange relient l'extérieur et l'intérieur du boitier, comme décrit plus en détail plus loin.

### Autres fonctions supportées par le dispositif

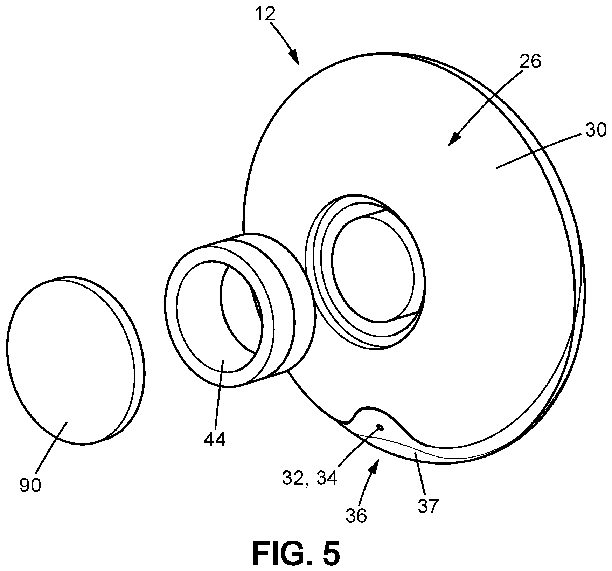

Dans l'exemple illustré à la figure 5, un capot amovible **90** est agencé sur la coque arrière. Le capot amovible permet de faciliter l'accès à l'ensemble de test **24.** Le capot amovible permet notamment de recharger le consommable **44** de l'ensemble de test **24.**

Le capot amovible **90** est ici fixé sur la coque arrière **30** par clipsage, vissage ou par un mécanisme de baïonnette. Bien entendu, d'autres moyens de fixation peuvent être mis en œuvre pour fixer le capot amovible sur la coque arrière **30.** Alternativement, dans un autre exemple, le capot amovible pourrait être fixé sur la coque avant **28.**

Le capot amovible **90** est agencé de manière étanche. Par exemple, une jointure entre le capot amovible et la coque arrière **30** peut comporter un joint d'étanchéité. Ainsi, l'intérieur du boitier **22** reste imperméable à l'urine, à l'eau provenant du réservoir d'eau **14** ou de la cuvette **16,** et tout autre type de contaminant.

Dans un autre exemple, le capot amovible 90 est formé par la coque avant 28 du boitier 22. Le capot amovible 90 peut alors être retiré par dévissage de la coque avant 28 par rapport à la coque arrière 30. Le boitier 22 comporte moins de jointures pouvant être salies et/ou infiltrées par l'eau des toilettes.

Le boitier **22** présente un orifice de collecte **32.** L'orifice de collecte peut recevoir l'urine ruisselant par gravité sur la surface extérieure du boitier **22.** Une collecte d'urine est réalisée directement depuis les faces **25, 26** du boitier.

L'orifice de collecte **32** est situé sur une extrémité inférieure **36** du boitier. L'extrémité inférieure **36** est orientée vers le fond de la cuvette **16** lorsque le boitier **22** est positionné dans la cuvette des toilettes. Cette position correspond à une position normale d'utilisation. Cette position permet une collecte de l'urine ruisselant par gravité sur la majorité de la surface extérieure du boitier.

En l'espèce, une distance **D** séparant l'orifice de collecte **32** d'une bordure inférieure 2**2**a du boitier **22** est inférieure à 40mm, de préférence inférieure à 20mm. Selon une réalisation particulière, l'orifice de collecte **32** est agencé à quelques millimètres au-dessus de la bordure inférieure du boîtier. En variante, l'orifice de collecte pourrait être sur la bordure inférieure **22a.**

L'orifice de collecte **32** est une ouverture, typiquement circulaire, de diamètre de préférence compris entre 0,3 mm et 2 mm. Le diamètre de l'orifice de collecte peut être choisi afin de maximiser le volume d'urine collecté de la surface extérieure du boitier.

Le boitier **22** présente un orifice de vidange **34.** L'orifice de vidange permet de purger le dispositif d'analyse d'urine d'un excès d'urine.

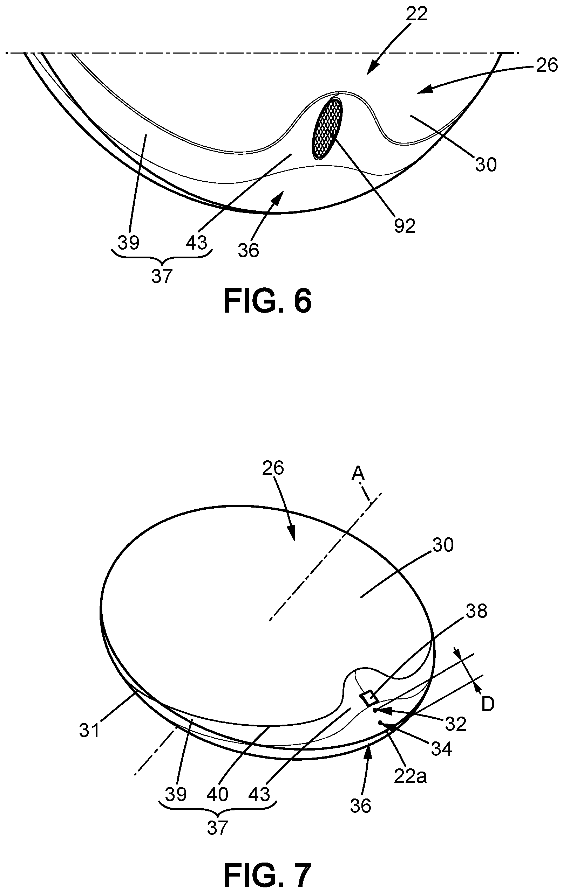

L'orifice de vidange **34** peut être distinct de l'orifice de collecte **32.** Alors, l'orifice de vidange est également situé sur l'extrémité inférieure du boitier **22,** au voisinage de l'orifice de collecte. L'orifice de vidange est également une ouverture circulaire. L'orifice de vidange **34** a un diamètre compris entre 0,3 mm et 2 mm. Dans la position normale d'utilisation, comme dans un mode de réalisation illustré sur la figure 7, l'orifice de vidange peut se trouver en dessous de l'orifice de collecte.

L'orifice de vidange 34 peut également être éloigné de l'orifice de collecte 32. La position de l'orifice de vidange 34 peut être choisie pour faciliter l'accès à l'orifice de vidange par l'ensemble de test 24. Par exemple, la figure 13 illustre un tel orifice de vidange 34. Il sera décrit plus en détail par la suite.

En variante, comme illustré à la figure 5, l'orifice de vidange **34** peut être le même que l'orifice de collecte **32.** Un unique orifice permet de limiter le nombre d'ouvertures vers l'intérieur du boitier **22.** Alors, le risque d'introduction de contaminants ou d'éléments susceptibles d'obstruer l'ensemble de test **24** est réduit.

Comme visible sur la figure 6, l'orifice de collecte **32** et l'orifice de vidange **34** peuvent être surmontés par un filtre **92** en maille métallique. Le filtre recouvre les orifices **32, 34.** Le filtre **92** en maille est par exemple de forme oblongue recouvrant les orifices **32,34.** L'ouverture moyenne de maille du filtre **92** est par exemple de 20 microns. Le filtre **92** permet d'empêcher l'introduction de contaminants ou d'éléments susceptibles d'obstruer l'ensemble de test **24,** et de filtrer l'urine reçue dans l'orifice de collecte **32.** Par ailleurs, le filtre **92** pourrait être nettoyé grâce à un flux d'air généré par une pompe à air. Dans une variante, seul l'orifice de collecte 32 est protégé par le filtre 92 en maille métallique. L'orifice de vidange 34, du fait de son agencement, de sa fonction et du débit qu'il éjecte, n'est pas de nature à contaminer l'orifice de collecte 32.

Dans les exemples illustrés aux figures 2 à 7 notamment, l'orifice de collecte **32** et l'orifice de vidange **34** sont situés sur la face arrière **26** du boitier. Alors, l'orifice de collecte et l'orifice de vidange font face à la paroi **16a** de la cuvette **16** lorsque le dispositif d'analyse d'urine **12** est positionné dans les toilettes **10.** Cette position permet de masquer l'orifice de collecte et l'orifice de vidange de la vue de l'utilisateur par la face avant **25** du boitier. La face avant visible de l'utilisateur ressemble à un simple galet uniforme comme déjà mentionné, sans aucun point singulier ni orifice ce qui confère un aspect un peu magique au dispositif d'analyse d'urine (d'apparence très simple, il produit des résultats très sophistiqués). Aussi, il faut noter que cette position permet d'empêcher l'introduction de contaminants ou d'éléments susceptibles d'obstruer l'ensemble de test **24.**

L'orifice de collecte **32** et l'orifice de vidange **34** sont situés dans un renfoncement **37.** Le renfoncement 37 est formé sur le boitier 22, en particulier sur la face arrière 26. Le renfoncement s'étend vers l'intérieur du boitier 22 sur une distance maximale comprise entre 1 et 4cm, par exemple entre 1,5cm et 3cm. Le renfoncement **37** comporte deux rainures latérales **39** s'étendant depuis la jointure **31** du boitier vers une partie centrale **43** du renfoncement 37 comportant les orifices **32, 34.** La profondeur des rainures latérales **39,** soit la distance depuis la face arrière **26** vers l'intérieur du boitier **22** selon la direction de l'axe **A,** croit depuis la jointure **31** jusqu'à la partie centrale **43.** Ainsi, le renfoncement **37** forme une amenée d'urine depuis la face avant **25** jusqu'à l'orifice de collecte **32.** Avantageusement, le renfoncement **37** permet de récolter de l'urine ruisselant sur la face avant **25** et l'acheminer vers l'orifice de collecte. Ainsi, le volume d'urine atteignant l'orifice de collecte depuis la face avant **25** du boitier est suffisant pour les besoins de l'analyse.

La partie centrale 43 du renfoncement 37, qui fonctionne comme un réservoir tampon, s'étend typiquement vers l'intérieur du boitier sur une distance comprises entre 1 et 4cm, par exemple entre 1,5cm et 3cm. Latéralement, ces mêmes dimensions de la partie centrale 43 sont applicables. Dans un mode de réalisation, la partie centrale 43 du renfoncement a une superficie d'au moins 3cm² et de moins de 8cm². De la sorte, le renfoncement 37 et en particulier la partie centrale 43 du renfoncement 37 crée un réservoir tampon (ouvert sur l'extérieur) pouvant recevoir de l'urine qui a ruisselé sur le dispositif, ce qui assure que l'orifice de collecte 32 reçoive de l'urine.

Le renfoncement 37 peut s'étendre sur toute la moitié inférieure du dispositif 12. En considérant que le dispositif 12 a une forme sphéroïde, il est possible de définir angulairement chaque point de la jointure 31 autour de l'axe A. En définissant l'orifice de collecte 32 comme le zéro angulaire, l'emplacement de la jointure 31 à partir duquel les rainures 39 se forment peut se situer à 90° (des deux côtés), ou entre 80 ou 90°. Alternativement, comme illustré en figure 13 par exemple, ledit emplacement peut être plus proche de l'orifice de collecte 32 et peut se situer entre 20° et 55°. La partie centrale 43 se trouve quant à elle à moins de 20°. Ainsi en fonction de dudit emplacement du point de départ des rainures 39 sur la jointure 31 (90°, 80°, 55° ou 20°, etc.), les rainures 39 sont plus ou moins longues.

Dans une variante illustrée en figure 13 par exemple, seul l'orifice de collecte 32 se trouve dans le renfoncement 37. L'orifice de vidange 34 se trouve sur la face arrière 26, en dehors du renfoncement 37 (entre 20 et 40°typiquement). En position normale d'utilisation du dispositif 12, l'orifice de vidange 34 se trouve donc plus haut que l'orifice de collecte 32. L'espacement entre les deux orifices 32, 34 permet de désencombrer le renfoncement 37. De plus, il a été observé que l'orifice de vidange 34 ainsi disposé ne génère pas de contamination de l'orifice de collecte 32.

Une bordure **40** délimitant le renfoncement **37** est arrondie. Autrement dit, la bordure **40** est définie par des surfaces courbées. La bordure **40** est dépourvue d'arête. De l'urine au contact de la face arrière **26** peut ruisseler vers l'orifice de collecte **32** sans décrocher du boitier ou former des bulles d'air. Ainsi, le volume d'urine atteignant l'orifice de collecte depuis la face arrière du boitier est augmenté. L'absence d'arête se vérifie le long de la bordure 40 (le long d'une ligne) et aussi orthogonalement à la bordure 40 (le long de lignes aussi). En d'autres termes, la surface tridimensionnelle définissant le renfoncement 37 (et incluant la bordure 40) ne présente pas d'arête. Cela signifie que la transition entre la face arrière 30 et la bordure 40 se fait sans arrête et que la transition entre la bordure et l'intérieur du renfoncement se fait sans arrête.

Dans un exemple de réalisation, l'orifice de collecte **32** est positionné 8 mm en dessous de la bordure **40.**

La bordure 40 du renfoncement 37 peut présenter une inflexion entre chaque rainure latérale 39 et la partie centrale 43, de sorte que, en suivant la bordure du renfoncement 37, la rainure latérale 39 est convexe (la convexité est définie du point de vue de la matière du dispositif et non pas de l'espace négatif complémentaire au dispositif), puis l'inflexion arrive et la partie centrale 43 est concave, puis une autre inflexion arrive et l'autre rainure latérale 39 est convexe. Alternativement, la bordure 40 du renfoncement 37 ne présente pas d'inflexion et demeure convexe.

Pour des raisons d'utilisation (i.e. le dispositif d'analyse d'urine 12 est disposé latéralement au milieu sur la paroi interne 16a de la cuvette 16a), le renfoncement 37 est typiquement symétrique. Toutefois, le renfoncement peut présenter une légère asymétrie pour favoriser une installation légèrement sur un côté gauche ou droit de la paroi interne 16a de la cuvette 16, et l'orifice de collecte 32 peut être légèrement positionné sur une partie droite ou gauche (pour qu'en utilisation l'orifice de collecte 32 soit quasiment centré latéralement sur la paroi interne 16a de la cuvette 16).

La distance entre la bordure 40 et un bord inférieur du dispositif 12 (c'est-à-dire la distance du segment intersectant la bordure 40 et le bord inférieur du dispositif, le segment étant orthogonal à la tangente audit bord au niveau de l'intersection) croit depuis l'endroit de la jointure **31** à partir duquel s'étend la rainure latérale (i.e. le début de la rainure latérale) jusqu'à la partie centrale **43.**

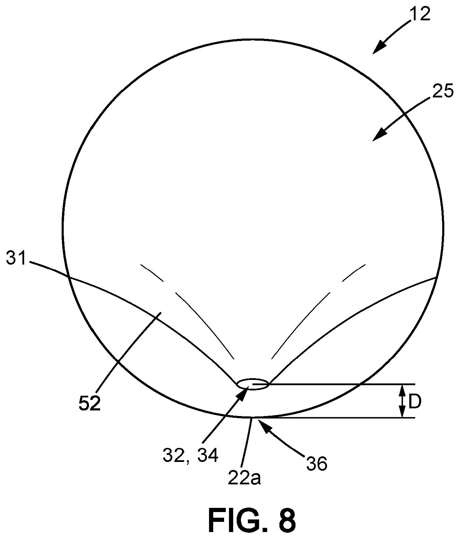

En variante, dans l'exemple illustré à la figure 8, l'orifice de collecte **32,** confondu avec l'orifice de vidange **34,** est situé sur la face avant **25** du boitier. Ainsi, l'urine ruisselant sur la face avant atteint l'orifice de collecte de manière plus directe.

Tel qu'illustré, l'orifice de collecte **32** est sur une saillie **52** de la face avant **25.** La saillie **52** s'étend depuis la jointure **31** vers l'extrémité inferieure **36** du boitier comportant l'orifice 32. La saillie forme également une amenée d'urine depuis la jointure **31** du boitier vers l'orifice de collecte **32.** La saillie permet de récolter de l'urine ruisselant sur la face avant **25** et l'acheminer vers l'orifice de collecte.

Il faut noter que l'orifice de collecte peut présenter une embouchure dirigée vers le haut, ou bien une embouchure dirigée vers le bas, sans exclure une embouchure dirigée purement radialement (c'est-à-dire à plat par rapport à la surface générale du boîtier).

Le boitier **22** ne se limite pas aux seuls modes de réalisation décrits ci-avant en regard des figures, mais est, au contraire, susceptible de nombreuses variantes accessibles à l'homme de l'art.

Le boitier **22** peut notamment prendre toute forme géométrique définie par des lignes courbes. Le boitier peut notamment avoir une forme de losange ou de goutte inversée. Alors, le boitier comporte une pointe sur la partie inférieure afin d'acheminer l'urine vers l'orifice de collecte **32.**

L'orifice de collecte **32** et l'orifice de vidange **34,** le cas échéant, peuvent être situés sur un relief positif, notamment une saillie, ou négatif, notamment une gouttière ou un renfoncement. De manière générale, le relief peut être de toute géométrie permettant de canaliser l'urine ruisselant sur le boitier **22** et de l'acheminer vers l'orifice de collecte **32** sans décrocher du boitier ou former des bulles d'air.

Dans un exemple de réalisation, l'orifice de collecte **32** est agencé sur la face avant **25,** alors que l'orifice de vidange **34** est situé sur la face arrière **26.**

### Ensemble de test

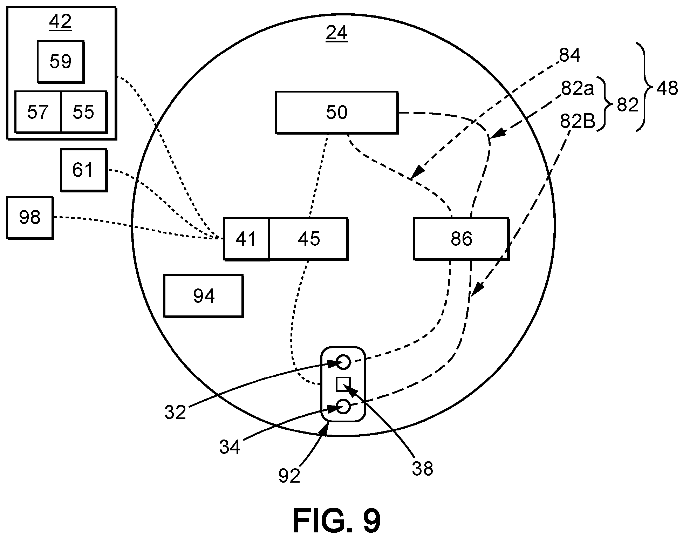

Par la suite, on décrit plus en détail l'ensemble de test **24,** en référence à la figure 9.

L'ensemble de test **24** est contrôlé par une unité électronique de commande **45.** L'unité de commande électronique 45 est à l'intérieur du boitier 22. L'unité électronique de commande **45** permet de contrôler les composants de l'ensemble de test pour réaliser une analyse d'urine et obtenir un ou plusieurs résultats d'analyses.

L'ensemble de test **24** comporte un système d'analyse **50.** Le système d'analyse **50** réalise l'analyse sur l'urine collectée depuis l'orifice de collecte **32** pour établir un ou plusieurs résultats. Ici, l'analyse peut être un ou plusieurs parmi :
- une analyse avec bandelettes colorimétriques, classiques ou du type immunodosage à flux latéral ou vertical : de l'urine est reçue sur une bandelette contenant un ou plusieurs réactifs sensibles à un ou plusieurs composants de l'urine, et une analyse par détection optique permet d'obtenir un résultat d'analyse ;
- une analyse avec une puce micro-fluidique, de type « lab on a chip » : la puce mélange de l'urine avec un ou plusieurs réactifs contenus dans des microgouttes, et une analyse par détection optique permettant d'obtenir un résultat d'analyse ;
- une analyse avec une puce micro-fluidique sur un support poreux : le mélange de l'urine avec un ou plusieurs réactifs est obtenu par capillarité ;
- une analyse avec un transistor à effet de champ, tel que décrit dans la demande de brevet publiée Withings EP3562407 ;
- une analyse par une mesure de conductivité de l'urine ;
- une analyse par une mesure de pH exploitant une sonde pH métrique ;
- une analyse par une mesure spectrophotométrique ;
- une analyse par une mesure électrochimique.

L'ensemble de test **24** comprend un moyen d'acheminement d'urine **48.** Le moyen d'acheminement d'urine **48** permet de d'acheminer de l'urine depuis l'orifice de collecte **32** jusqu'à l'orifice de vidange **34,** au travers le système d'analyse **50.** De manière préférée, le moyen d'acheminement d'urine 48 comporte un canal de collecte **82,** un canal de purge **84** et une pompe **86.**

Le canal de collecte **82** relie l'orifice de collecte **32** au système d'analyse 50. Le canal de collecte **82** permet d'acheminer de l'urine depuis l'orifice de collecte **32** vers le système d'analyse **50.**

Le canal de purge **84** relie le système d'analyse **50** à l'orifice de vidange **34.** Le canal de purge permet notamment une évacuation de l'urine contenue dans le moyen d'acheminement d'urine **48.** De préférence, le canal de purge 84 est hydrophobe, permettant une meilleure évacuation de l'urine. Alors, le risque de contamination de l'urine acheminé entre deux collectes successives est réduit.

La pompe **86** est agencée entre une première portion **82a** et une deuxième portion **82b** du canal de collecte **82.**

La pompe **86** peut aspirer de l'urine depuis l'orifice de collecte **32.** La pompe **86** aspire par exemple entre 5 microlitres et 1 mL, de préférence environ 20 microlitres. Alors, la pompe permet d'acheminer un volume suffisant d'urine pour réaliser une analyse concluante. Un débit d'aspiration de la pompe est choisi en fonction du diamètre de l'orifice de collecte. Alors, la pompe peut aspirer de l'urine depuis l'orifice de collecte vers le système d'analyse **50** sans former de bulles d'air.

La pompe **86** peut également aspirer de l'air depuis l'orifice de collecte **32.** L'air traverse le moyen d'acheminement d'urine **48** jusqu'à l'orifice de vidange **34** pour évacuer l'urine contenue dans le moyen d'acheminement d'urine. L'urine collectée pour une analyse est alors protégée d'une éventuelle contamination par une collecte précédente.

En variante, la pompe **86** pourrait aspirer de l'eau lors de l'activation de la chasse d'eau des toilettes **10** pour évacuer l'urine contenue dans le moyen d'acheminement d'urine **48.**

La pompe **86** est ici une pompe de type piézoélectrique. Par l'usage d'une pompe piézoélectrique, le dispositif d'analyse d'urine peut être dépourvu de valves contrôlées, ce qui simplifie le dispositif d'analyse d'urine.

En variante, la pompe peut être un système pneumatique. Alors, le système pneumatique est configuré pour créer une pression négative afin d'aspirer l'urine depuis l'orifice de collecte, et une pression positive pour pousser l'urine vers le système d'analyse 50 et l'orifice de vidange. Cette solution permet de contrôler de manière précise le volume collecté par le dispositif d'analyse d'urine 12.

L'ensemble de test 24 comporte un capteur de présence d'urine 38. Le capteur de présence d'urine 38 est agencé à proximité de l'orifice de collecte 32. Le capteur de présence d'urine permet alors de détecter lorsque de l'urine est présent au voisinage de l'orifice de collecte.

Selon un mode de réalisation, le capteur de présence d'urine 38 pourrait former un anneau autour de l'orifice de collecte. L'intégration du capteur de présence d'urine dans le dispositif d'analyse d'urine est alors discrète.

De manière préféré, le capteur de présence d'urine **38** est un capteur de température, par exemple une thermistance. Le capteur de température permet en effet de distinguer entre de l'urine et de l'eau provenant des toilettes 10. En outre, le capteur de température peut également être exploité pour mesurer la température de l'urine. La température de l'urine permet notamment de détecter des périodes de fécondité. L'utilisation d'un capteur de température permet alors de réduire le nombre de composants exploités par l'ensemble de test **24** pour réaliser une analyse. La complexité et les coûts liés à la fabrication du dispositif d'analyse d'urine sont réduits.

Alternativement, le capteur de présence d'urine **38** peut être tout type de détecteur de liquide, par exemple un capteur de type capacitif ou résistif. Alors, un capteur de température est distinct du capteur de présence d'urine. Le capteur de température peut être dédié à la mesure de la température de l'urine, notamment pour détecter une période de fécondité.

### Communication et aspects système

Le dispositif d'analyse d'urine comporte un module de communication **41.** Le module de communication **41** est sans-fil. Le module de communication 41 exploite un réseau local, par exemple de type Bluetooth, Low-Energy Bluetooth (BLE) ou Wi-fi. Le réseau local permet de préserver une batterie **94** de l'ensemble de test 24. Ainsi, l'autonomie du dispositif d'analyse **12** est augmentée.

En variante, le module de communication **41** peut exploiter un réseau de télécommunication cellulaire. Le réseau de télécommunication cellulaire peut par exemple être GSM, 3G, 4G, 5G,4G-LTE. Le module de communication 41 a alors une portée plus longue.

Alternativement encore, le module de communication **41** peut exploiter une passerelle connectée au réseau de télécommunication cellulaire. La passerelle peut notamment être un routeur, par exemple un routeur Wi-fi connecté au réseau cellulaire, un hub, c'est-à-dire un dispositif connecté directement au réseau cellulaire, ou le smartphone de l'utilisateur. Alors, le dispositif d'analyse d'urine 12 peut être connecté au réseau cellulaire sans compromettre l'autonomie de la batterie 94.

De préférence, le module de communication 41 exploite la technologie Bluetooth Low Energy (BLE) pour communiquer avec un smartphone 61 de l'utilisateur et la technologie Wifi pour se connecter à un serveur distant 98.

Le module de communication **41** permet de déclencher une analyse via une commande à distance.

De manière préférée, l'analyse est lancée depuis le smartphone **61** de l'utilisateur. L'utilisateur peut lancer une analyse depuis une application sur le smartphone. L'utilisateur a le contrôle sur le dispositif d'analyse d'urine 12, et peut choisir lorsqu'il souhaite réaliser une analyse. L'utilisateur peut également être identifié au travers l'utilisation du smartphone. L'analyse réalisée peut alors être adaptée à l'utilisateur. L'utilisateur peut également sélectionner l'analyse qu'il souhaite réaliser.

De manière alternative, l'analyse est lancée par communication avec un appareil déporté **42** à proximité des toilettes. L'appareil déporté comporte un bouton **55.** L'utilisateur peut alors appuyer sur le bouton pour lancer une analyse. L'utilisateur a le contrôle sur le dispositif d'analyse d'urine, et peut choisir lorsqu'il souhaite réaliser une analyse.

Le bouton **55** peut être pourvu d'un capteur biométrique **57.** Le bouton peut alors identifier l'utilisateur appuyant sur le bouton. Alors, une analyse pertinente à l'utilisateur identifié peut être effectuée par le dispositif d'analyse d'urine. En outre, l'analyse effectuée peut être choisie en fonction de l'utilisateur identifié, et les résultats envoyés pour enrichir l'historique de cet utilisateur identifié.

L'appareil déporté **42** comporte également un afficheur **59.** Par exemple, l'afficheur peut consister en une ou plusieurs diodes électroluminescentes (LED) de couleurs. L'afficheur peut également comprendre un écran. L'afficheur permet d'informer l'utilisateur. Par exemple, l'utilisateur peut être informé qu'un appui sur le bouton a été perçu, et/ou que l'utilisateur a été identifié et/ou qu'une analyse va être réalisée.

Alternativement, l'appareil déporté **42** peut être un bracelet connecté associé à l'utilisateur. Dans ce cas, l'utilisateur peut être automatiquement détecté lorsqu'il est à proximité des toilettes. L'utilisateur peut également être identifié par le bracelet connecté. Ainsi, une analyse peut être lancée automatiquement, sans action de l'utilisateur. À noter que le bracelet connecté peut se présenter sous la forme d'une montre connectée.

Le module de communication **41** permet également de communiquer le ou les résultats d'analyses. Le ou les résultats d'analyses peuvent être un ou plusieurs parmi : une période de fécondité, une grossesse, des infections urinaires, des problèmes de foie, des insuffisances rénales, une lithiase urique, une déshydratation, une maladie cardiaque et/ou d'un diabète. Le ou les résultats peuvent également être un indicateur de l'observance médicamenteuse.

Le module de communication **41** peut envoyer le ou les résultats d'analyses directement à l'afficheur 59 ou au smartphone 61. Le module de communication peut alors être dépourvu d'une connexion au réseau de télécommunication cellulaire. Le ou les résultats d'analyses peuvent être interprétés localement par l'unité électronique de commande 45, ou par l'application du smartphone. Ainsi, les coûts d'exploitation du dispositif d'analyse d'urine 12 sont réduits.

Alternativement, le module de communication **41** peut envoyer le ou les résultats vers un serveur **98** distant. Le serveur distant 98 peut interpréter le ou les résultats. L'exploitation d'un serveur permet de réduire les capacités de calcul nécessaire localement pour l'interprétation du ou des résultats d'analyses.

Le serveur distant 98 peut également être pourvu d'une capacité de stockage. Ainsi, le serveur distant peut stocker les ou les résultats d'une pluralité d'analyses successives.

L'utilisateur peut visualiser et exploiter un ou plusieurs résultats, reçu directement du dispositif d'analyse 12 ou du serveur disant. Par exemple, l'utilisateur peut visualiser et exploiter les résultats depuis l'application du smartphone. Alternativement, l'utilisateur peut accéder à un site web depuis un ordinateur.

Il peut être prévu que le boitier **22** comprenne un ou plusieurs indicateurs lumineux (par exemple des diodes électroluminescentes, i.e des LEDs) agencés à l'intérieur du boîtier en vis-à-vis d'une portion translucide de la face avant du boîtier de sorte à pouvoir ainsi fournir à l'utilisateur un retour visuel sur le fonctionnement du dispositif d'analyse 12. Il peut être prévu plusieurs couleurs et des motifs de clignotement pour signifier à l'utilisateur divers statuts du dispositif.

Enfin, un bouton 192 peut être accessible à l'utilisateur depuis le boitier 22. Le bouton 192 est accessible à l'utilisateur pour réinitialiser le dispositif d'analyse d'urine 12. Le bouton 192 peut notamment être accessible à l'utilisateur lorsque le capot amovible 90 est retiré.

### Unité de commande électronique

De préférence, l'unité de commande est construite par un système sur puce. Une première puce Bluetooth Low Energy peut prendre en charge la commande des composants électronique du dispositif d'analyse d'urine. La première puce BLE peut également prendre en charge la communication avec le dispositif à proximité. Une deuxième puce Wifi peut prendre en charge l'échange de données avec le serveur distant. Ainsi, le deuxième système sur puce peut être éteint lorsqu'une communication avec le serveur n'est pas utilisée. La consommation d'énergie de l'unité de commande est améliorée, et la consommation de la batterie s'en trouve réduite.

L'unité de commande peut également être divisée en différents circuits imprimés. Chaque circuit imprimé peut commander différents composants électronique du dispositif d'analyse d'urine. Un circuit principal peut assurer la coopération entre les différents circuits imprimés. Une telle construction permet une flexibilité quant à la construction de l'unité de commande électronique et son intégration dans le boitier.

L'unité de commande électronique 45 est alimentée par la batterie 94 prévue à l'intérieur du boitier 22. La batterie 94 est de type lithium-ion. La capacité de la batterie est d'environ 1080mAh. Une telle capacité permet d'assurer une autonomie satisfaisante du dispositif sans compromettre les dimensions du boitier.

La batterie 94 comprend des connecteurs de charge 54. Comme notamment visible en figure 13, les connecteurs de charge 54 sont accessibles depuis l'extérieur du boitier 22 pour permettre une recharge de la batterie 94. Par exemple, le boitier 22 peut être placé sur un socle pour relier les connecteurs de charge 54 à une source d'énergie. Alternativement, une recharge par induction pourrait également être envisagée.

### Aspects de procédé

Par la suite, on décrit plus en détail deux procédés permettant de lancer une analyse d'urine et de recevoir le ou les résultats. Les procédés sont mis en œuvre par l'unité de commande électronique **45.**

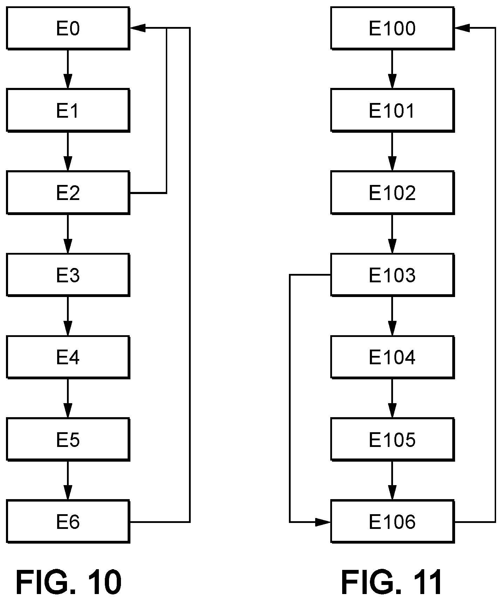

Dans l'exemple illustré à la figure 10, le procédé est initié par interaction avec l'utilisateur.

A l'étape **E0,** le dispositif d'analyse d'urine 12 est dans un état inactif. Dans l'état inactif, le dispositif d'analyse d'urine attend de recevoir une demande d'analyse.

Alors, l'étape **E1** correspond la réception de la demande d'analyse. La demande d'analyse peut provenir de l'appui sur le bouton **55** par l'utilisateur. La demande d'analyse peut également être commandée depuis le smartphone 61. La demande d'analyse peut également être réalisée automatiquement, lorsque l'utilisateur est à proximité des toilettes 10. L'afficheur 59 peut indiquer à l'utilisateur que son appui a été reçu. L'afficheur peut également indiquer si un utilisateur a été reconnu lors de l'appui sur le bouton.

A l'étape **E2,** le dispositif d'analyse d'urine 12 entre dans un état actif. A l'état actif, le dispositif d'analyse d'urine attend de détecter de l'urine à proximité de l'orifice de collecte 32. Le boitier 22 peut en outre être équipé de LEDs pour alerter un utilisateur que le dispositif d'analyse d'urine est dans le mode actif.

Si aucun jet d'urine n'est reçu dans un délai imparti, alors le dispositif d'analyse d'urine 12 retourne à l'état inactif de l'étape **E0.** Si, au contraire, un jet d'urine est détecté, la pompe 86 est activée pour acheminer l'urine depuis l'orifice de collecte 32 vers le système d'analyse 50 (Etape **E3).**

L'étape **E4** consiste à réaliser une analyse sur l'urine collectée. L'analyse réalisée dépend du dispositif d'analyse 50 dans le boitier 22. L'analyse réalisée peut également dépendre de l'utilisateur identifié par le bouton 55. L'analyse réalisée peut également dépendre du choix effectué par l'utilisateur.

L'étape **E5** correspond à la transmission du ou des résultats. Le ou les résultats peuvent être transmis directement à l'utilisateur. Le ou les résultats peuvent également être envoyés vers le serveur 98. L'utilisateur peut par exemple visualiser et exploiter le ou les résultats sur l'application du smartphone 61, ou sur un site web. Le ou les résultats peuvent également être envoyés vers un professionnel de santé.

Enfin, l'étape **E6** consiste à activer la pompe 86 pour purger le dispositif d'analyse d'urine 12 d'un excès d'urine. Le dispositif d'analyse d'urine retourne par la suite à l'état inactif de l'étape **E0.**

En variante, dans l'exemple illustré à la figure 11, le procédé est initié lorsque de l'urine est détecté sur le boitier 22.

A l'étape **E100,** le dispositif d'analyse d'urine 12 est à l'état inactif. Ici, l'état inactif correspond à l'attente de détection d'urine à proximité de l'orifice de collecte 32.

Alors, l'étape **E101** consiste à détecter la présence d'urine à proximité de l'orifice de collecte.

A l'étape **E102,** la pompe **86** est activée pour aspirer l'urine depuis l'orifice de collecte vers le système d'analyse **50.** L'afficheur **59** peut indiquer qu'une analyse est prête à être réalisée. En variante, le boitier **22,** pourvu de LEDs, peut indiquer qu'une analyse est prête à être réalisée.

A l'étape **E103,** le dispositif d'analyse d'urine 12 attend la réception d'une demande d'analyse d'un utilisateur. La demande d'analyse peut provenir de l'appui sur le bouton **55** par l'utilisateur. La demande d'analyse peut également être commandée depuis le smartphone **61.**

Si la demande d'analyse n'est pas reçue dans un délai imparti, alors la pompe **86** est activée pour purger le dispositif d'analyse d'urine **12** de l'urine collectée (Etape **E106).** Le dispositif d'analyse d'urine retourne à l'état inactif de l'étape **E100.**

Si au contraire la demande d'analyse est reçue, alors une analyse est réalisée, à l'étape **E104.** En outre, l'afficheur **59** peut indiquer à l'utilisateur que son appui a été reçu. L'afficheur peut également indiquer si un utilisateur a été reconnu lors de l'appui sur le bouton **55.**

L'étape **E105** correspond à la transmission du ou des résultats. Le ou les résultats peuvent être transmis directement à l'utilisateur. Le ou les résultats peuvent également être envoyés vers le serveur **98.** L'utilisateur peut par exemple visualiser le ou les résultats sur l'application du smartphone **61,** ou sur un site web. Le ou les résultats peuvent également être envoyés vers un professionnel de santé.

Enfin, l'étape **E106** consiste à activer la pompe 86 pour purger le dispositif d'analyse d'urine 12 d'un excès d'urine. Le dispositif d'analyse d'urine retourne par la suite dans le mode inactif de l'étape **E100.**

## Revendications

1. Dispositif d'analyse d'urine (12) comprenant un boitier (22) configuré pour être entièrement positionné à l'intérieur de toilettes (10),
le boitier présentant une face avant (25) destinée à recevoir un jet d'urine directement d'un utilisateur urinant sur les toilettes, une face arrière (26) opposée à la face avant, et un orifice de collecte (32), et
dans lequel le boitier contient un ensemble de test (24) configuré pour effectuer une analyse sur l'urine collectée par l'orifice de collecte,
dans lequel la face arrière (26) est configurée pour être orientée face à une paroi intérieure (16a) d'une cuvette (16) des toilettes, et
**caractérisé en ce que** l'orifice de collecte (32) est disposé sur la face arrière (26).

2. Dispositif d'analyse d'urine (12) selon la revendication 1, dans lequel le boitier (22) est dépourvu d'arête.

3. Dispositif d'analyse d'urine (12) selon la revendication 1 ou 2, dans lequel la face avant (25) et la face arrière (26) sont reliées par des bords courbés (27).

4. Dispositif d'analyse d'urine (12) selon l'une quelconque des revendications précédentes, configuré pour collecter de l'urine par l'orifice de collecte (32) directement par ruissellement sur les faces du boitier (22).

5. Dispositif d'analyse d'urine (12) selon l'une quelconque des revendications précédentes, dans lequel la face arrière (26) comprend un relief (37,43) formant une amenée d'urine vers l'orifice de collecte (32).

6. Dispositif d'analyse d'urine (12) selon la revendication 5, dans lequel le relief est positif, par exemple de type saillie.

7. Dispositif d'analyse d'urine (12) selon la revendication 5 ou 6, dans lequel le relief est négatif, par exemple de type renfoncement.

8. Dispositif d'analyse d'urine (12) selon l'une quelconque des revendications 5 à 7, dans lequel le relief est formé dans le boitier.

9. Dispositif d'analyse d'urine (12) selon l'une quelconque des revendications 1 à 8, dans lequel l'orifice de collecte (32) est situé au voisinage d'une extrémité inferieure (36) du boitier (22) en position normale d'utilisation.

10. Dispositif d'analyse d'urine (12) selon l'une quelconque des revendications 1 à 9, dans lequel le boitier (22) a une forme générale de galet circulaire.

11. Dispositif d'analyse d'urine (12) selon l'une quelconque des revendications 1 à 10, dans lequel le diamètre du boitier (22) est compris entre 50 mm et 150 mm, de préférence le diamètre du boitier est de 100 mm.

12. Dispositif d'analyse d'urine (12) selon l'une quelconque des revendications 1 à 11, comprenant en outre un capteur de présence d'urine (38) au voisinage de l'orifice de collecte (32), le capteur étant configuré pour détecter la présence d'urine, de préférence le capteur étant un capteur de température.

13. Dispositif d'analyse d'urine (12) selon l'une quelconque des revendications 1 à 12, comprenant un outre un module de communication (41) avec un appareil (42) et/ou un smartphone (61) et/ou un serveur (98).

14. Dispositif d'analyse d'urine (12) selon l'une quelconque des revendications 1 à 13, dans lequel l'ensemble de test (24) comporte un ou plusieurs parmi : une pluralité de bandelettes de test, une puce microfluidique, un transistor à effet de champ, un dispositif de mesure de conductivité, un dispositif de mesure de pH, un dispositif de mesure spectroscopique, un dispositif de mesure électrochimique.

15. Méthode de collection d'urine exploitant le dispositif d'analyse d'urine (12) selon l'une quelconque des revendications 1 à 14, comprenant :
- détecter la présence d'urine à proximité de l'orifice de collecte (32),
- collecter l'urine ruisselant sur la face arrière (26),
- analyser l'urine collectée pour établir un ou plusieurs résultats d'analyses.

## Patentansprüche

1. Urinanalysevorrichtung (12), die ein Gehäuse (22) umfasst, das so konfiguriert ist, dass es vollständig innerhalb einer Toilette (10) positioniert werden kann,
wobei das Gehäuse eine Vorderseite (25) zum Empfangen eines Urinstrahls direkt von einem Benutzer, der auf die Toilette uriniert, eine Rückseite (26), die der Vorderseite gegenüberliegt, und eine Sammelöffnung (32) aufweist, und
wobei das Gehäuse eine Testanordnung (24) enthält, die so konfiguriert ist, dass sie eine Analyse des durch die Sammelöffnung gesammelten Urins durchführt,
wobei die Rückseite (26) so konfiguriert ist, dass sie einer Innenwand (16a) eines Toilettenbeckens (16) zugewandt ist, und
**dadurch gekennzeichnet, dass** die Sammelöffnung (32) auf der Rückseite (26) angeordnet ist.

2. Urinanalysevorrichtung (12) nach Anspruch 1, wobei das Gehäuse (22) keine Kanten aufweist.

3. Urinanalysevorrichtung (12) nach Anspruch 1 oder 2, bei der die Vorderseite (25) und die Rückseite (26) durch gekrümmte Kanten (27) verbunden sind.

4. Urinanalysevorrichtung (12) nach einem der vorhergehenden Ansprüche, die so konfiguriert ist, dass sie Urin durch die Sammelöffnung (32) direkt durch Abtropfen auf die Seiten des Gehäuses (22) sammelt.

5. Urinanalysevorrichtung (12) nach einem der vorhergehenden Ansprüche, wobei die Rückseite (26) eine Erhebung (37, 43) aufweist, die eine Urinzuführung zur Sammelöffnung (32) bildet

6. Urinanalysevorrichtung (12) nach Anspruch 5, bei der das Relief positiv ist, z. B. vom Typ Vorsprung.

7. Urinanalysevorrichtung (12) nach Anspruch 5 oder 6, bei der das Relief negativ ist, z. B. vom Typ Vertiefung.

8. Urinanalysevorrichtung (12) nach einem der Ansprüche 5 bis 7, wobei das Relief in dem Gehäuse ausgebildet ist.

9. Urinanalysevorrichtung (12) nach einem der Ansprüche 1 bis 8, wobei die Sammelöffnung (32) in der normalen Gebrauchsposition in der Nähe eines unteren Endes (36) des Gehäuses (22) angeordnet ist.

10. Urinanalysevorrichtung (12) nach einem der Ansprüche 1 bis 9, wobei das Gehäuse (22) die allgemeine Form einer kreisförmigen Rolle hat.

11. Urinanalysevorrichtung (12) nach einem der Ansprüche 1 bis 10, wobei der Durchmesser des Gehäuses (22) zwischen 50 mm und 150 mm beträgt, vorzugsweise beträgt der Durchmesser des Gehäuses 100 mm.

12. Urinanalysevorrichtung (12) nach einem der Ansprüche 1 bis 11, ferner umfassend einen Urinanwesenheitssensor (38) in der Nähe der Sammelöffnung (32), wobei der Sensor so konfiguriert ist, dass er die Anwesenheit von Urin detektiert, wobei der Sensor vorzugsweise ein Temperatursensor ist.

13. Urinanalysevorrichtung (12) nach einem der Ansprüche 1 bis 12, die zusätzlich ein Modul (41) zur Kommunikation mit einem Gerät (42) und/oder einem Smartphone (61) und/oder einem Server (98) umfasst.

14. Urinanalysevorrichtung (12) nach einem der Ansprüche 1 bis 13, wobei die Testanordnung (24) eines oder mehrere von Folgendem umfasst: eine Vielzahl von Teststreifen, einen Mikrofluidik-Chip, einen Feldeffekttransistor, eine Leitfähigkeitsmessvorrichtung, eine pH-Messvorrichtung, eine spektroskopische Messvorrichtung, eine elektrochemische Messvorrichtung.

15. Verfahren zum Sammeln von Urin, das die Urinanalysevorrichtung (12) nach einem der Ansprüche 1 bis 14 ausnutzt, umfassend :
- das Vorhandensein von Urin in der Nähe der Sammelöffnung (32) feststellen,
- den auf die Rückseite (26) rieselnden Urin sammeln,
- den gesammelten Urin analysieren, um ein oder mehrere Analyseergebnisse zu erstellen.

## Claims

1. A urine analysis device (12) comprising a case (22) configured to be positioned entirely within a toilet (10),
the case having a front face (25) for receiving a stream of urine directly from a user urinating on the toilet, a rear face (26) opposite the front face, and a collection port (32), and
wherein the case contains a test assembly (24) configured to perform an analysis on urine collected through the collection port,
wherein the rear face (26) is configured to face an inner wall (16a) of a toilet bowl (16), and
**characterised in that** the collection orifice (32) is disposed on the rear face (26).

2. The urine analysis device (12) according to claim 1, wherein the case (22) has no ridge.

3. The urine analysis device (12) according to claim 1 or 2, wherein the front face (25) and the rear face (26) are connected by curved edges (27).

4. The urine analysis device (12) according to any one of the preceding claims, configured to collect urine through the collection orifice (32) directly by trickling onto the faces of the case (22).

5. The urine analysis device (12) according to any one of the preceding claims, wherein the rear face (26) comprises a relief (37, 43) forming a pathway for urine towards the collection orifice (32).

6. The urine analysis device (12) according to claim 5, wherein the relief is positive, for example of the projecting type.

7. The urine analysis device (12) according to claim 5 or 6, wherein the relief is negative, for example of the recess type.

8. The urine analysis device (12) according to any one of claims 5 to 7, wherein the relief is formed in the case.

9. The urine analysis device (12) according to any one of claims 1 to 8, wherein the collection orifice (32) is located in the vicinity of a lower end (36) of the case (22) in the normal position of use.

10. The urine analysis device (12) according to any one of claims 1 to 9, wherein the case (22) has the general shape of a circular roller.

11. The urine analysis device (12) according to any one of claims 1 to 10, wherein the diameter of the case (22) is between 50 mm and 150 mm, preferably the diameter of the case is 100 mm.

12. The urine analysis device (12) according to any one of claims 1 to 11, further comprising a urine presence sensor (38) in the vicinity of the collection orifice (32), the sensor being configured to detect the presence of urine, preferably the sensor being a temperature sensor.

13. The urine analysis device (12) according to any one of claims 1 to 12, further comprising a communication module (41) with an apparatus (42) and/or a smartphone (61) and/or a server (98).

14. The urine analysis device (12) according to any one of claims 1 to 13, wherein the test assembly (24) comprises one or more of: a plurality of test strips, a microfluidic chip, a field effect transistor, a conductivity measuring device, a pH measuring device, a spectroscopic measuring device, an electrochemical measuring device.

15. A method of collecting urine using the urine analysis device (12) according to any one of claims 1 to 14, comprising:
- detect the presence of urine near the collection port (32),
- collect the urine dripping onto the rear face (26),
- analyse the urine collected to establish one or more analysis results.
